# EUROPÄISCHE PATENTSCHRIFT

(11) **EP 1 715 879 B1**
(45) Veröffentlichungstag und Bekanntmachung des Hinweises auf die Patenterteilung: **11.04.2007**
(21) Anmeldenummer: 05701191.8
(22) Anmeldetag: 26.01.2005
(51) Int. Cl.: A61K 36/8962

(54) **ZWIEBELEXTRAKTE**
ONION EXTRACTS
EXTRAITS D'OIGNONS

(30) Priorität: 27.01.2004 DE 102004004034
(43) Veröffentlichungstag der Anmeldung: 02.11.2006
(73) Patentinhaber: Divapharma Chur AG, 7006 Chur (CH)
(72) Erfinder: EICH, Jürgen, 98744 Cursdorf (DE); GOERKE, Thomas, 10956 Berlin (DE); HAFFNER, Thomas, 65187 Wiesbaden (DE)
(74) Vertreter: Gesthuysen, von Rohr & Eggert
(86) Internationale Anmeldenummer: PCT/EP2005/000750
(87) Internationale Veröffentlichungsnummer: WO 2005/070441

(56) Entgegenhaltungen:
- US-A1- 2002 187 207

## Beschreibung

Die Erfindung betrifft Extrakte aus Zwiebeln *(Allium cepa* L.) und Verfahren zu deren Herstellung sowie deren Verwendung in verschiedenen Anwendungsgebieten.

### STAND DER TECHNIK

Zwiebeln (*Allium cepa* L.) werden weltweit in großen Mengen kultiviert und sind in vielen Ländern der Erde wichtiger Bestandteil der Nahrung. Die zu einer Zwiebel verdickten fleischigen Blattscheiden und Blattansätze von *A. cepa* werden in der Regel roh oder verarbeitet als Gemüse und Gewürz verwendet. Zwiebeln und Zwiebelzubereitungen werden daneben auch traditionell als Arzneimittel zur äußerlichen Behandlung von Insektenstichen, infizierten Wunden, Verbrennungen usw. sowie zur innerlichen Behandlung bei Husten, Bronchitis, Asthma, Störungen im Magen-Darm-Trakt, Bluthochdruck und Atherosklerose verwendet [Monographie Allii cepae bulbus, in: Hänsel R, Keller K, Rimpler H, Schneider G (Hrsg.): Hager's Handbuch der Pharmazeutischen Praxis. 5. Aufl., Band 4: Drogen A-D. Springer Verlag, Berlin, Heidelberg, New York 1992: 184-187 und die dort zitierten Arbeiten]. So ist z.B. die Verwendung von Zwiebeln und deren Zubereitungen als Mittel zur Behandlung von Appetitlosigkeit und zur Vorbeugung von altersbedingter Gefäßveränderung mit antibakteriellen, lipid- und blutdrucksendenden Eigenschaften z.B. in Deutschland offiziell anerkannt [Monographie *Allii cepae bulbus* (Zwiebel) der Kommission E des BGA, Bundesanzeiger 50 vom 13.03.1986]. Von der Monographie wird eine tägliche Einnahmemenge an Zwiebeln oder entsprechenden Zubereitungen zur Erreichung der therapeutischen Wirksamkeit empfohlen, die 50 g frischen oder 20 g getrockneten Zwiebeln entspricht. Zur Anwendung kommen dabei Zubereitungen wie Pflanzenöl-Extrakte (Mazerate), Trockenpulver oder ätherisches Öl-Zubereitungen in entsprechenden Darreichungsformen. So gibt es z.B. in Deutschland ca. 40 behördlich verzeichnete Arzneimittel, wovon nur wenige vermarktet werden - allesamt ohne besondere wirtschaftliche Bedeutung. Der Grund hierfür dürfte in der Tatsache liegen, daß die von der Monographie empfohlene tägliche Einnahmemenge an Zwiebeln relativ hoch ist (z.B. ca. 40 Hartgelatinekapseln mit 500 mg Zwiebelpulver). Für die verfügbaren Zubereitungen sind die einzunehmenden Mengen zum Erreichen der empfohlenen Tagesdosis ebenfalls zu groß. Dadurch ist eine arzeiliche Anwendung bei Verbrauchern (Patienten) bisher unattraktiv geblieben.

Frische Zwiebeln enthalten im wesentlichen Wasser (ca. 80-90%), verschiedene Zucker wie Fructane, reduzierende Zucker und Saccharose (ca. 5%), die für den Geschmack und Geruch verantwortlichen schwefelhaltigen Verbindungen und phenolische Verbindungen wie z.B. Flavonoide. Die oben beschriebene Wirksamkeit der Zwiebel wird im wesentlichen auf den Gehalt an schwefelhaltigen Verbindungen und an Flavonoiden zurückgeführt [BREU W. und DORSCH W., Economic and Medicinal Plant Research 1994; 6: 115-147].
Von den bekannten Gemüsearten enthalten Zwiebeln (gelbe und rote) die höchsten Gehalte des Flavonoids Quercetin, vorwiegend in Form seiner Glukoside [HERRMANN K., Z. Lebensm. Unters. Forsch. 1988; 186: 1-5; LEIGHTON T. *et al.* in: Food and Cancer Prevention: Chemical and Biological Aspects 1989; 87 223-232; PARK Y.K. und LEE C.Y., Polyphenols 1994, Palma de Mallorca, May 23-27, INRA (eds.), Paris 1995, Les Colloques n°69]. Im Gegensatz zu anderen Flavonoiden besitzt Quercetin eine Reihe gesundheits-förderlicher (Schutz und Vorbeugung) und therapeutisch nutzbarer Eigenschaften (arzneiliche Wirkungen), welche durch pharmakologische und klinische Studien belegt sind [Monographie Allii cepae bulbus, in: Hänsel R, Keller K, Rimpler H, Schneider G (Hrsg.): Hager's Handbuch der Pharmazeutischen Praxis. 5.

Aufl., Band 4: Drogen A-D. Springer Verlag, Berlin, Heidelberg, New York 1992: 184-187 und die dort zitierten Arbeiten]. Quercetin, welches aus Quercetin-Derivat reichen Drogen wie Zwiebeln oder Buchweizen durch Extraktion und Hydrolyse zu gewinnen ist, ist eine der am stärksten antioxidativ wirksamen natürlich vorkommenden phenolischen Verbindungen, welche oxidative Schädigungen von Zellmembranen und LDL-Partikeln durch Radikale in vitro verhindert sowie den antioxidativen Status lebender Organismen nach Einnahme signifikant verbessert. Daneben wurden positive Wirkungen zur Vorbeugung und Therapie von Herz-Kreislauf-Erkrankungen, chronisch entzündlichen Darmerkrankungen, Krebserkrankungen, Allergien und Entzündungen, Diabetes und Virusinfektionen gefunden [GRÖBER U., PTA heute 2001; 4: 48-52; GRÖBER U., PTA heute 2001; 5: 54-56; FORMICA, J.V. und REGELSON, W., Fd. Chem. Tox. 1995; 33: 1061-1080].

Eine prophylaktische und therapeutische Wirksamkeit bei den meisten der oben genannten Anwendungsgebiete setzt die gastro-intestinale Resorption ausreichender Mengen einer Verbindung voraus - man spricht von systemischer Verfügbarkeit oder Bioverfügbarkeit. Von reinem Quercetin (Flavon-Aglykon) ist bekannt, daß es aufgrund seiner schlechten Wasserlöslichkeit nur eine sehr geringe Bioverfügbarkeit besitzt. Auch einige seiner Glykosid-Derivate wie zum Beispiel das Rutin (Quercetin-3-O-rutosid), welches in Buchweizen in hoher Konzentration vorkommt, oder die Galaktoside, Arabinoside, Rhamnoside und Xyloside des Quercetins in Äpfeln werden nach Einnahme nur schlecht resorbiert [HOLLMAN P.C.H. et *al.,* FEBS Lett. 1997; 418: 152-156]. Eine Ausnahme bilden die beiden wichtigsten Quercetin-Glukoside der Zwiebel, Quercetin-4'-O-glukosid (= Spiraeosid (im folgenden auch als QMG bezeichnet) und Quercetin-3,4'-di-O-glukosid (im folgenden auch als QDG bezeichnet), welche um das 2- bis 3-fache besser bioverfügbar sind als das ungebundene Quercetin bzw. die oben genannten anderen Glykoside. Beide Verbindungen kommen nur in gelben und roten Zwiebeln in höheren Konzentration vor (100 - 1700 mg/kg Frischgewicht = 0,01 - 0,17 % der frischen Zwiebel). Im frischen Teil der Zwiebel nehmen beide Glucoside einen Anteil von ca. 85 - 95 % an den gesamten Flavonoiden ein, während freies Quercetin hier nur in Spuren vorkommt (< 2 %). Die trockenen äußeren Schalen der gelben und roten Zwiebeln enthalten im Gegensatz dazu nur einen relativ kleinen Anteil des Quercetin-diglukosids sowie hohe Anteile an Quercetinmonoglukosid und freiem Quercetin. Die gute Bioverfügbarkeit der beiden obigen Glukoside wird in Verbindung gebracht mit der jeweiligen stereoselektiven Bindung der Glucose an der 4'-O-, bzw. 3-O-Position des Quercetins, welche eine Glukose-Transporter vermittelte Aufnahme der Quercetin-Derivate im oberen Dünndarmbereich ermöglicht [beispielhaft: PRICE K.P. und RHODES M.J.C., J. Sci. Food Agric. 1997; 74:331-339; HOLLMAN P.C.H. *et al.,* FEBS Lett. 1997; 418: 152-156; HOLLMAN P.C.H. und KATAN M.B., Food and Chemical Toxicology 1999; 37: 937-942; ADER P. *et al.,* Free Rad. Biol. Med. 2000; 28: 1056-1067; HOLLMAN P.C.H. *et al.* Free Rad. Res. 1999; 31: 569-573; GEE J.M. *et al*., Free Radical Biology & Medicine 1998; 25: 19-25].

Zwiebeln enthalten neben den beschriebenen Verbindungen auch die unerwünschte Verbindung Diphenylamin. Für Diphenylamin ist eine hypoglykämische (blutzuckersenkende) Wirkung beschrieben [Karawya M.S. *et al*., Egypt. J. Pharm. Sci. 1984; 25: 21-25; Karawya M.S. und Wahab S.M.A., J. Natural Prod. 1984; 47: 775-780; Smoczkiewiczowa A. *et al.,* Herba Polonica 1990; 3: 97-109]. Aus diesem Grund ist der maximale Gehalt dieser Verbindung in Zwiebeln und deren Zubereitungen indirekt durch die maximal zulässige Tagesaufnahme zur arzneilichen Anwendung von der Monographie limitiert worden [Monographie Allii cepae bulbus (Zwiebel) der Kommission E des BGA, Bundesanzeiger 50 vom 13.03.1986].

In der wissenschaftlichen und Patentliteratur sind Extraktionsmethoden zur Gewinnung von Flavonoid-haltigen Extrakten aus Zwiebeln und anderen Pflanzen dokumentiert. Die folgenden Beispiele in den Tabellen 1 A und B zeigen typische allgemeine und technische Methoden zur Extraktion der Flavonoide aus Zwiebeln bzw. Pflanzenmaterial:

**Tabelle 1: Allgemeine und technische Extraktionsmethoden zur Gewinnung von Extrakten bzw. Flavonoid-haltigen Extrakten aus Zwiebeln bzw. anderem Pflanzenmaterial**

| **Nr.** | **Extraktions mittel** | **Zwiebel/ Material** | **Bedingungen** | **Referenz** |
|---|---|---|---|---|
| **Tabelle 1A: Allgemeine Verfahren** | | | | |
| 1 | Methanol (100 %) | frisch | einmal bei Raumtemperatur; Verhältnis Extraktionsmittel/ Zwiebel 4 : 1 (m/m) | TSUSHIDA T. und SUZUKI M., Nippon Shokuhin Kagaku Kogaku Kaishi 1996; 43: 642-649 |
| 2 | Methanol (100 %) | frisch | einmal bei Raumtemperatur; Verhältnis Extraktionsmittel/ Zwiebel 40:1 (m/m) | BILYK A. et al., J. Agric. Food Chem. 1984; 32: 274-276 |
| 3 | Methanol (100%) | getrocknet | einmal bei Raumtemperatur; Verhältnis Extraktionsmittel/ Zwiebel ? | SUH H.J. et al., Food Res. Intern. Food Res. Intern. 1999; 32: 659-664 |
| 4 | Methanol (80%) | frisch | einmal bei 0 - 4 °C; Verhältnis Extraktionsmittel/ Zwiebel 8:1 (m/m) | HIROTA S. *et al.,* J. Agric. Food Chem. 1998; 46: 3497-3502 |
| 5 | Methanol (70%) | Gefriergetrocknet | 3-fach bei Raumtemperatur; Verhältnis Extraktionsmittel/ Zwiebel 25:1 (m/m) | PRICE K.R. *et al.*, J.Agric. Food Chem. 1997; 45: 938-942 |
| 6 | Ethanol | frisch | nicht bekannt | YANG X *et al.,* Natural Medicines 1999; 53: 149 |
| 6a | Ethanol (80%) | | nicht bekannt | Patil *et al.* J. Horticultural Sci. 1995; 70: 643-650 |
| 7 | Butanol + Essigsäure (0,75 %) | frisch | einmal bei 4 ° C für 18 h; Verhältnis Extraktionsmittel/Zwiebel 1:1 (m/m) | LEIGTHON T. *et al*., ACS Symp. Ser. 1992; 507: 220-238 |

| **Tabelle 1B: Technische Verfahren** | | | | |
|---|---|---|---|---|
| 8 | mehrstufige Extraktion mit Chloroform, Aceton, Wasser | unbekannt | Vorextraktion mit Chloroform, Extraktion mit Aceton, verdünnen mit Wasser, Filtration, verbliebener Rückstand wird in 50 % Ethanol kristallisiert. Ausbeute an Quercetin: 2,6 % (Glukoside: ?) | Patent SU 483981 |
| 9 | einfache Extraktion mit Ethanol 85-95% | frisch | 1-fache Extraktion mit Ethanol bei 40 °C, Filtration | Patent FR 2681531 |
| 10 | Ethanol | frisch | 1-fache Extraktion mit Ethanol | Patent JP 7327636 |
| 11 | Wasser, Alkohol | frisch | Zwiebel mit Mikrowelle bestrahlen, tieffrieren, kalt mit Wasser oder wässrigen kurzkettigen Alkoholen extrahieren, gefriertrocknen | Patent EP 592382* |
| 12 * | mehrstufige Extraktion mit Aceton, Wasser, nicht polaren Lösungsmitt Lösung, Alkohol, Essig-, Ameisensäure | Getrocknet | Vorextraktion mit Aceton/Wasser-Gemisch, Entfernung von fettlöslichen Bestandteilen mit apolarem Lösungsmittel, Extraktion der a) alkalisch gemachten Phase mit Alkohol, b) der sauren Phase mit Alkohol, Aufkonzentrierung im Vakuum, Zugabe eines Gemisches aus Methanol, Wasser und Essig (Ameisen-) säure, Aufkonzentrierung, Kristallisation* | Patent KR 9402797* |
| 13 * | 2-stufige Extraktion mit wässrigem Puffer und organische Lösungsmittel | unbekannt | Vorextraktion mit gepuffertem Wasser, Re-Extraktion mit organischem Lösungsmittel | Patent KR 9402798 |
| 14 | Wasser | frisch | Heißextraktion mit Wasser | BELITZ H.-D. *et al.,* Lehrbuch der Lebensmittelchemie; Springer Verlag, Berlin-Heidelberg, 5. Aufl. 2001; HEISS R., Lebensmitteltechnologie. Biotechnologische, chemische, mechanische und thermische Verfahren der Lebensmittelverarbeitung; Springer Verlag, Berlin-Heidelberg, 5. Aufl. 2001 |

| | | | | |
|---|---|---|---|---|
| * diese Verfahren sind für die Gewinnung von Flavonglykosiden aus verschiedenen Pflanzen beschrieben | | | | |

Für keinen der mit den oben aufgeführten Verfahren hergestellten Zwiebelextrakte ist die qualitative und quantitative Zusammensetzung bezüglich ihres Gehalts an Flavonoiden bekannt. Aus diesem Grund wurden die wichtigsten Extraktionsversuche von den gegenwärtigen Erfindern nachgearbeitet (Ergebnisse in Tabelle 2A)

**Tabelle 2A: Vergleich verschiedener Auszugsbedingungen/mittel zur Herstellung von Flavonoid-angereicherten Zwiebelextrakten nach Stand der Technik (vergleiche Tabelle 1)**

| **Extrakt mit** | **Gesamt gehalt Quercetin- Derivate** | **Verhältnis QDG/ QMG** | **Droge/ Extrakt - Verhältnis *** | **% extrahierte Querce tin-derivate** | **Gehalt Diphenylamin %** | **Wertung technischer Verarbeitbarkeit **** | **Wertung typischer Zwiebelgeruch und Geschmack ***** |
|---|---|---|---|---|---|---|---|
| Frischzwiebel | 0,90 | 1,50 | entfällt | entfällt | 1,07 | entfällt | entfällt |
| 100%-Methanol | 1,34 | 1,50 | 1,5 | 93 | 0,40 | schlecht | stark |
| 70%-Methanol | 1,34 | 1,52 | 1,4 | 100 | 0,11 | schlecht | stark |
| 100%-Aceton | 4,08 | 0,59 | 24,1 | 18 | 3,18 | mäßig | stark |
| 100%-Ethylacetat | 1,00 | 0,11 | 94,6 | 1 | 3,65 | schlecht | mittel |
| 100 %-Ethanol | 1,92 | 1,49 | 2,4 | 84 | 0,67 | schlecht | stark |
| Butanol + Essigsäure 133 +1 (V/V) | 0,55 | 0,81 | 10,4 | 6 | 0,20 | gut | schwach |
| 60 % wässr. Aceton | 1,40 | 1,50 | 1,4 | 100 | 0,39 | schlecht | schwach |
| 83 % wässr. Butanol | 4,23 | 1,27 | 5,3 | 100 | 0,11 | gut | mittel |

| | | | | | | | |
|---|---|---|---|---|---|---|---|
| * das Droge/Extrakt-Verhältnis ist das Verhältnis der Massen der zur Extraktion eingesetzten Droge zum daraus erhaltenen Extrakt. ** die Verarbeitung wurde durch die die Verarbeitung durchführende Person bewertet. *** der Geruchs- und Geschmackstest wurde mit Hilfe von sechs Testpersonen durchgeführt, deren subjektive Bewertungen als "schwach", "mittel", "stark" gemittelt wurden. | | | | | | | |

Alle bekannten Verfahren, dokumentiert in Tabelle 1 und 2 besitzen für den Fall der Gewinnung von Quercetin-Glukosidreichen Extrakten aus der Zwiebel mindestens einen oder gleich mehrere der im folgenden genannten Nachteile:
1. Zu geringer Gehalt an Quercetin-Glulcosiden im Extrakt (< 5 %) :
   durch eine zu geringe Selektivität des Verfahrens/Auszugsmittels bezüglich der Extraktion der Glukoside (zu viele unerwünschte Begleitstoffe werden mit extrahiert z.B. Zucker, dadurch steigt die benötigte Menge an Extrakt für sinnvolle Tagesdosen)
2. Verlust der nativen Zusammensetzung des Mono-/Diglucosids:
   das Verfahren/Auszugsmittel ist zu selektiv bezüglich des Mono- und/oder Diglucosids und kann nur eines der beiden Glukoside vollständig extrahieren (mangelhafte Ausbeute) [z.B. bei Nr. 6, 9, 11, 13, 14 aus Tabelle 1]
3. Hohe Anteile des Aglycons Quercetin:
   durch Extraktion tritt die unerwünschte chemische oder enzymatische Hydrolyse der Glukoside auf - das Verfahren ist nicht schonend genug, bzw. die Glukosid-spaltenden Enzyme wurden nicht inhibiert [z.B. bei Nr. 7, 12-14 aus Tabelle 1]
4. Komplexes, aufwendiges, kostenintensives technisches Verfahren:
   i.d.R. durch Mehrstufenextraktion - technische Umsetzung ist unwirtschaftlich [z.B. bei Nr. 8, 11-13 aus Tabelle 1]
5. Hoher Diphenylamingehalt:
   hohes Toxizitätspotential - mangelnde Eignung der Extrakte zum Einsatz als Lebensmittel (Giftigkeit) oder Arzneimittel (Nebenwirkungspotentiale) [z.B. bei allen Extrakten aus Tabelle 1]
6. Mangelhafte technische Verarbeitbarkeit der gewonnenen Extrakte
   i.d.R. durch Mitextraktion klebriger Begleitstoffe [z.B. bei Nr. 1-6, 9-11, 13-14 aus Tabelle 1]
7. Starker, störender Zwiebelgeruch:
   geringe Verbraucherakzeptanz [z.B. bei Nr. 1-6, , 9-14 aus Tabelle 1]

Bevorzugte Verfahren sollten für die Herstellung kommerziell nutzbarer Extrakte mindestens folgende Eigenschaften haben:
a) Extrakt mit hohen Gehalten an Quercetin-4'-O-glukosid (QMG) und Quercetin-3,4'-di-O-glukosid (QDG) (zusammen mindestens 10 % im Extrakt) gewonnen aus frischen oder getrockneten Zwiebeln (A. cepa).
b) Weitgehend unveränderte, native Zusammensetzung der beiden Glukoside der frischen, bzw. getrockneten Zwiebel (Verhältnis QDG/QMG = 0,5 - 1,5)
c) geringer Gehalt an Quercetin (Aglykon)
d) Gehalt an Diphenylamin an oder unter der analytischen Nachweisgrenze von 0,01 %
e) Einfaches, kostengünstiges Herstellverfahren
f) einfache technische Verarbeitbarkeit der gewonnenen Extrakte
g) verringerter Zwiebelgeruch

Es ist daher eine Aufgabe der vorliegenden Erfindung, einen Extrakt bereitzustellen, der die oben genannten Eigenschaften aufweist.

Diese Aufgabe wurde erfindungsgemäß wie in den unabhängigen Ansprüchen beschrieben gelöst. Vorteilhafte Ausführungsformen sind in den abhängigen Ansprüchen angegeben

### ZUSAMMENFASSUNG DER ERFINDUNG

Insbesondere betrifft die Erfindung einen Extrakt aus frischen oder getrockneten Zwiebeln (Allium cepa L.), enthaltend: mindestens 10 %, vorzugsweise 20 - 60 % Quercetin und/oder Quercetin-Derivate. Die Erfindung betrifft ferner ein Verfahren zur Herstellung eines Extraktes aus Zwiebeln gemäß, umfassend die Schritte:
- Extraktion frischer oder getrockneter Zwiebeln mit einem organischen Lösungsmittel aus der Reihe der Alkohole mit Ausnahme von Methanol und Ethanol, Ketone, Ester, Ether, insbesondere mit 1-, 2-Butanol, Aceton, Ethyl-, und/oder Methylacetat.
- Gewinnen der organischen Phase.

Beansprucht wird auch die Verwendung der Extrakte zur Herstellung von Arzneimitteln, Nahrungsmitteln, Nahrungsergänzungsmitteln, diätetischen Lebensmitteln und Kosmetika. Diese können erfindungsgemäß verwendet werden für die Behandlung und Prophylaxe von Entzündungen, insbesondere Nieren-, Blasen-, Harnwegs- und Prostataentzündungen im akuten und chronischen Stadium, Gelenkentzündungen und rheumatische Erkrankungen im akuten und chronischen Stadium, Herz/Kreislauferkrankungen und Atherosklerose, Diabetes mellitus begleitenden Erkrankungen, insbesondere Kataraktbildung, Neuropathien und Nierenschädigungen, Demenzerkrankunge, Parkinson'scher Krankheit und Alzheimer'scher Krankheit.

### AUSFÜHRLICHE BESCHREIBUNG DER ERFINDUNG

Aufgabe war es, alle oben aufgeführten Nachteile bisheriger Verfahren und Extraktqualitäten zu überwinden.

Die Erfindung beruht auf dem überraschenden Befund, daß kommerziell nutzbare Extrakte aus Allium cepa mit sehr hohen Quercetin(derivat)-Gehalten im Gegensatz zu allen bisher gekannten Verfahren erhalten werden, wenn erfindungsgemäß die frischen oder getrockneten Zwiebeln mit einem organischen Lösungsmittel aus der Reihe der Alkohole, Ketone, Ester, Ether, insbesondere mit 1-, 2-Butanol, Aceton, Ethyl-, und/oder Methylacetat, insbesondere n-Butanol, Aceton, extrahiert werden. Gemäß einer bevorzugten Ausführungsform umfasst das oben genannte Extraktionsverfahren die folgenden nachgeschalteten Schritte:
- Waschen der organischen Phase mit Wasser und
- Gewinnen der organischen Phase.

Noch bevorzugter umfasst es die folgenden nachgeschalteten Schritte:
- Waschen des Extraktes mit Wasser,
- Gewinnen und Verdampfen der organischen Phase,
- Extraktion des Rückstands mit einem unpolarem Lösungsmittel aus der Reihe der Alkane, Alkene, Ether, Ketone, Ester, insbesondere mit n-, iso-Hexan, n-, iso-Heptan, Methyl-tert.-butylether, Aceton und/oder überkritischem Kohlendioxid,
- Verwerfen der flüssigen organischen Phase bzw. des Extraktes, gewonnen mit überkritischem Kohlendioxid und
- Trocknen des Rückstands.

Insbesondere umfasst es die folgenden nachgeschalteten Schritte:
- Waschen des Extraktes mit Wasser,
- Gewinnen, Einengen oder Verdampfen der organischen Phase,
- Umlösung des Rückstandes in Wasser,
- Extraktion des gelösten Rückstands mit einem organischen Lösungsmittel aus der Reihe der Alkane, Alkene, Ether, Ketone, Ester, insbesondere n-, iso-Hexan, n-, iso-Heptan, Methyl-tert.-butylether, Aceton und/oder überkritischem Kohlendioxid und
- Abtrennung und Verwerfen der organischen Phase.

Zur Vorbereitung der Extraktion werden gemäß einer weiteren bevorzugten Ausführungsform frische Zwiebeln zerkleinert (1 - 20 mm Teilchengröße) oder auf Teilchengrößen von 1 - 10 mm geschnittene trockene Zwiebeln schnellstmöglich (weniger als 1 h) mit dem organischen Extraktmittel in einer Extraktionsapparatur intensiv in Kontakt gebracht. Dabei wird entweder ein Ultra-Turrax oder eine ähnliche Zerkleinerungsvorrichtung oder ein Ultraschallgeber verwendet. Möglich ist auch eine intensive Extraktion in einem Gegenstrom-Extraktor. Die Extraktion wird unter Normaldruck bei Temperaturen zwischen 30 und 70 °C bis zur Erschöpfung durchgeführt. Nach Extraktion wird die native Extraktmischung zur Phasentrennung für einige Stunden bei 4-8 °C gekühlt. Es können auch andere phasentrennende Verfahrensschritte wie z.B. Zentrifugation angewendet werden.

Anschließend werden organische und wässrige Phase abtrennt, die organische getrocknet und zur Gewinnung des Trockenextraktes verdampft. Hierbei können Vakuumbandtrockner oder Dünnschichtverdampfer oder Konvektionsöfen verwendet werden.

Die erfindungsgemäß hergestellten Extrakte weisen überraschenderweise gegenüber dem Ausgangsmaterial und Extrakten nach dem Stand der Technik folgende Vorteile auf:
1. Einstufiges Herstellverfahren (nur ein organisches Auszugsmittel)
2. Gehalt Quercetinglucoside: 10 bis 60 %, insbesondere 20-60%
3. Verhältnis QDG/QMG: 0,5 - 2,0 %
4. Gehalt an Quercetin-Aglykon: <4 %, insbesondere < 2 %
5. Gehalt Diphenylamin: <0,1 %, insbesondere < 0,01 %
6. Gute, direkte technische Verarbeitbarkeit
7. Verringerter Zwiebelgeruch

Dies ist auch Tabelle 2B zu entnehmen, die die Eigenschaften der erfindungsgemäßen Extrakte im Vergleich zum Stand der Technik darstellt (s. unten). Die Extrakte können erfindungsgemäß zur Herstellung einer Zubereitung mit antioxidativer, zell-, gefäß-, gewebs- und organprotektiver Wirkung verwendet werden.

Der erfindungsgemäße Extrakt kann beispielsweise in festen pharmazeutischen Darreichungsformen, Nahrungsergänzungsmitteln und diätetischen Lebensmitteln wie Tabletten, Filmtabletten, Dragees, Kautabletten, Brausetabletten oder Pulver zur Anwendung formuliert werden. Dabei können die typischen Hilfsstoffe, wie (derivatisierte) Cellulosen, Milchzucker, Talkum, Zerfallsbeschleuniger, Siliciumdioxid, Saccharose, Polymethacrylate, PEGs, Citronensäure, Natriumhydrogencarbonat, Magnesiumstearat, Farbstoffe, Geschmacksstoffe etc. verwendet werden.

Der erfindungsgemäße Extrakt kann ebenso in halbfesten und flüssigen Formen zur pharmazeutischen und kosmetischen Anwendung und zur Anwendung als Nahrungsergänzungsmittel und diätetische Lebensmittel formuliert werden. Typische Formen sind Lösungen, Tropfen, Weichgelatinekapseln, Gele, Salben, Cremes, Lotionen, Puder etc. Dabei können die jeweils typischen Hilfsstoffe Verwendung finden: z.B. Wasser, Ethanol, Lösungsvermittler, Gelbildner, Fettphasen, Träger, Farbpigmente, Geruchs- und Geschmackskorrigenzien.

### BEISPIELE

Im folgenden wird die Erfindung durch Beispiele im Detail beschrieben. Sie sollte jedoch nicht als darauf begrenzt angesehen werden.

| Beispiel Filmtablette (Mengen pro Tablette) | |
|---|---|
| 300 mg | Zwiebelextakt |
| 349 mg | Lactose |
| 40 mg | Cellulose |
| 20 mg | Natriumcrosscarmellose |
| 18 mg | Siliciumdioxid |
| 4 mg | Mg-Stearat |
| 25 mg | Hydroxypropylmethylcellulose |
| 10 mg | Polyethylenglykol (PEG) |
| 2 mg | Farbstoff |
| 3 mg | Titandioxid |

| Beispiel Creme | |
|---|---|
| 15 kg | Glycerylstearat |
| 22 kg | Cetylalcohol |
| 28 kg | Neutralöl |
| 96 kg | Vaseline |
| 0,8 kg | Vitamin E |
| 10 kg | Zwiebelextrakt |
| 26 kg | PEG-20 Glycerylstearat |
| 38 kg | 1,2-Propylenglycol |
| 182 kg | Wasser |

In Tabelle 2B sind die Eigenschaften der erfindungsgemäßen Extrakte im Vergleich dargestellt:

| | | | | | | | |
|---|---|---|---|---|---|---|---|
| Erfindungs gemäße Extrakte | >10 | 0,5 - 1,5 | 20 | 100 | < 0,01 | sehr gut | schwach |

### Die Parameter wurden wie folgt bestimmt:

### Bestimmung des Gehalts an Quercetin-Derivaten

Vorbereitung: Frische Zwiebeln, von denen die äußeren trockenen Schichten abgeschält worden waren, wurden geschnitten und nach dem Einfrieren bei -60°C gefriergetrocknet. Getrocknete Zwiebeln wurden pulverisiert. Zwiebel- oder Extraktpulver wurde in Meßkolben eingewogen (50 bis 100 mg), mit 80 % MeOH gefüllt und 30 min im Ultraschallbad behandelt. Die Suspension wurde zentrifugiert, der Überstand mit Wasser 1:1 verdünnt zur Injektion verwendet (20µl).

Analytik: Die Trennung erfolgte an einem Waters-HPLC-System mit Suplcosil LC-18-Säule 150 x 4,6 mm und einem Acetonitril-Phosphorsäure-Gradienten bei einer Flußrate von 1 ml/min. Die Detektion erfolgte bei 270 nm. Im Chromatogramm von Zwiebelproben treten Quercetin-3,4'-di-O-glucosid und Quercetin-4'-glukosid in etwa vergleichbaren Mengen auf. Weiterhin können zwei Di- und drei Monoglukoside sowie freies Quercetin detektiert werden [s. auch Tsushida *et al.*, Nippon Shokuhin Kagaku Kogaku Kaishi 1996; 43: 642-649]. Die Quantifizierung der Quercetin-Derivate erfolgte über einen externen Referenzstandard bekannten Gehalts (Quercetin-4'-glukosid).

### Trockenextrakt aus frischen Zwiebeln

Geschälte Zwiebeln werden mit Hilfe eines geeigneten Gerätes (z.B. Küchenmaschine, Ultraturrax) in einem organischen Lösemittel, vorzugsweise n-Butanol zerkleinert und für 3 - 5 min bei Raumtemperatur zu einem Mus homogenisiert. Das Verhältnis Auszugsmittel zu Frischmasse beträgt 0,5-4:1, vorzugsweise 1-2:1. Zur Abtrennung des Zwiebelrückstandes wird die Suspension zügig filtriert oder zentrifugiert.

Anschließend läßt man die organische Oberphase des Filtrates bzw. des Überstandes absetzen. Nach Abtrennen der wässrigen Unterphase wird die Oberphase einmal mit Wasser gewaschen. Die gewaschene Oberphase wird anschließend zur Trockene eingeengt. Das vorhandene organische Restlösemittel kann durch erneutes Auflösen des Extraktes in Wasser und anschließende Trocknung entfernt werden. Der Trockenextrakt weist einen Gehalt an Quercetin-Derivaten von mindestens 10%, vorzugsweise von 20 bis 60 % auf.

### Trockenextrakt aus getrockneten Zwiebeln

Getrocknetes Material von geschälten Zwiebeln wird mit Hilfe eines geeigneten Gerätes (z.B. Mixer, Ultraturrax) in einem organischen, Lösemittel, vorzugsweise n-Butanol zerkleinert und für 3 - 5 min bei Raumtemperatur zu einem Mus homogenisiert. Das Verhältnis Auszugsmittel zu Masse getrockneter Zwiebel beträgt 2-25:1, vorzugsweise 7-14:1. Zur Abtrennung des Zwiebelrückstandes wird die Suspension filtriert oder zentrifugiert. Der Filtrat- bzw. Zentrifugenüberstand wird mit Wasser gewaschen. Die gewaschene organische Oberphase wird anschließend zur Trockene eingeengt. Das vorhandene Restlösemittel kann durch erneutes Auflösen des Extraktes in Wasser und anschließende Trocknung entfernt werden. Zur Entfernung eventuell vorhandener, störender lipophiler Verbindungen kann der Extrakt mit einem unpolaren Lösungsmittel gewaschen und anschließend erneut getrocknet werden.

### Beispiel 1

7500 g frische Zwiebel ohne äußere braune Schalen (enthalten 990 mg Quercetin-Derivate pro kg Frischmasse), wurden in 7,5 1 n-Butanol in einem Mixer fein zerkleinert und sofort filtriert. Das Filtrat wurde zur Phasentrennung einige Stunden auf 5 °C gekühlt. Die Oberphase wurde abgetrennt und mit einem Fünftel ihres Volumens an Wasser gewaschen. Die gewaschene Oberphase wurde zur Trockene eingeengt. Es wurden 18,9 g Trockenextrakt erhalten. Der Trockenextrakt weist einen Gehalt an Quercetin-Derivaten von 25,9 % auf.

### Beispiel 2

500 g frische Zwiebel ohne äußere braune Schalen (enthalten 1930 mg Quercetin-Derivate pro kg Frischmasse) werden in 0,5 1 n-Butanol in einem Mixer fein zerkleinert und sofort filtriert. Das Filtrat wird zur Phasentrennung einige Stunden auf 5 °C gekühlt. Die Oberphase wird abgetrennt und mit einem Fünftel ihres Volumens an Wasser gewaschen. Die gewaschene Oberphase wird zur Trockene eingeengt. Es werden 1,36 g Trockenextrakt erhalten. Der Trockenextrakt weist einen Gehalt an Quercetin-Derivaten von 38,8 % auf.

### Beispiel 3

150 g getrocknete Zwiebel ohne äußere trockene braune Schalen (enthalten 0,44% Quercetin-Derivate) werden in 1,1 1 n-Butanol in einem Mixer fein zerkleinert und sofort filtriert. Das Filtrat (organische Phase) wird zur Klärung einige Stunden auf 5 °C gekühlt, und mit einem Fünftel seines Volumens an Wasser gewaschen. Die gewaschene Oberphase wird zur Trockene eingeengt. Es werden 2,88 g Trockenextrakt erhalten. Der Trockenextrakt weist einen Gehalt an Quercetin-Derivaten von 16,14 % auf.

### Beispiel 4

150 g getrocknete Zwiebel ohne äußere trockene braune Schalen (enthalten 1,99 % Quercetin-Derivate) werden in 1,1 1 n-Butanol in einem Mixer fein zerkleinert und sofort filtriert. Das Filtrat (organische Phase) wird zur Klärung einige Stunden auf 5 °C gekühlt, und mit einem Fünftel seines Volumens an Wasser gewaschen. Die gewaschene Oberphase wird zur Trockene eingeengt. Es werden 5,74 g Trockenextrakt erhalten. Der Trockenextrakt weist einen Gehalt an Quercetin-Derivaten von 40,72 % auf.

### Beispiel 5

### Analyse des Diphenylamingehalts

### Verfahren

Das Diphenylamin wird durch Zerkleinern der frischen Zwiebeln im Mixer unter Zugabe von Wasser herausgelöst. Danach wird das Diphenylamin aus einem Teil der Probenlösung durch flüssig/flüssig Extraktion mit Chloroform an Diatomeenerde extrahiert.

Von dieser Probenlösung werden 1µl splitlos in das GC/MS-System injiziert. Die Zuordnung des Diphenylamins erfolgt über die Retentionszeit und das charakteristische Isotopencluster; die Ermittlung der Konzentration über externe Standardisierung.

| Geräteparameter | | |
|---|---|---|
| Gaschromatograph | HP5890 Serie II Agilent | |
| Temperaturprogramm | 50°C (1 min, 25 °C/min) | |
| | 150°C (0 min, 10 °C/min) | |
| | 200 °C (0 min, 30 °C/min) | |
| | 320 °C (3 min, 17 °C/min) | |
| Massenspektrometer | SSQ 7000 | Thermo-Finnigan |
| Injektionssystem | KAS | Gerstel GmbH |
| Autosampler | A200S | Thermo Finnigan |
| GC-Kapillarsäule | HP-5 30m; 0.32 mm id ; | 0.25µm Film |
| | | Agilent |
| Software | Xcalibur Revision 1.1 | Thermo Finnigan |

### Aufbereitung der Probe

Es werden 2 g frische Zwiebeln mit 100 ml Wasser im Ultra Turax 10 min behandelt. 20 ml dieser Lösung werden über eine 20 ml Kartusche mit Diatomeenerde geschüttet. Nachdem die Probe eingelaufen ist, wird fünf Minuten gewart et. Anschließend wird mit dreimal je 10 ml Chloroform in einen 25 ml Meßkolben nachgespült (extrahiert). Der Meßkolben wird auf 25 ml aufgefüllt. Von dieser Lösung werden 1µl splitlos injiziert.

### Berechnung Gehalt Diphenylamin

Die Bestimmung des Gehalts an Diphenylamin erfolgte über eine Dreisatzberechnung durch Vergleich einer Referenzlösung mit reiner Referenzsubstanz.

### Beispiel 6

Beschreibung der Verfahren für die Ausarbeitung der Tabelle 2 50 - 100 mg Extrakte werden in einem Meßkolben eingewogen, mit 80 % MeOH gefüllt und 30 min im Ultraschallbad behandelt. Die Suspension wurde zentrifugiert, der Überstand mit Wasser 1:1 verdünnt zur Injektion verwendet (20µl).

Die Trennung erfolgte an einem Waters-HPLC-System mit Suplcosil LC-18-Säule 150 x 4,6 mm und einem Acetonitril-Phosphorsäure-Gradienten bei einer Flußrate von 1 ml/min. Die Detektion erfolgte bei 270 nm. Im Chromatogramm von Zwiebelproben treten Quercetin-3,4'-di-O-glucosid und Quercetin-4'-glukosid in etwa vergleichbaren Mengen auf. Weiterhin können zwei Di- und drei Monoglukoside sowie freies Quercetin detektiert werden [s. auch Tsushida et *al.,* Nippon Shokuhin Kagaku Kogaku Kaishi 1996; 43: 642-649]. Die Quantifizierung der Quercetin-Derivate erfolgte über einen externen Referenzstandard bekannten Gehalts (Quercetin- 4'-glukosid).

## Patentansprüche

1. Extrakt aus frischen oder getrockneten Zwiebeln (Allium cepa L.), enthaltend: mindestens 10 %, vorzugsweise 20 - 60 % Quercetin und/oder Quercetin-Derivate, erhältlich durch ein Verfahren, umfassend die Schritte:
- Extraktion frischer oder getrockneter Zwiebeln mit einem organischen Lösungsmittel aus der Reihe der Alkohole mit Ausnahme von Methanol und Ethanol, Ketone, Ester, Ether, insbesondere mit 1-, 2-Butanol, Aceton, Ethyl-, und/oder Methylacetat,
- Gewinnen der organischen Phase.

2. Extrakt gemäß Anspruch 1, enthaltend: mindestens 10 %, vorzugsweise 20 - 60 % Quercetin-Glukoside.

3. Extrakt gemäß Anspruch 1 oder 2 mit einem Verhältnis von Quercetin-3,4'-di-O-glukosid zu Quercetin-4'-O-glukosid von 0,5 - 2.

4. Extrakt gemäß Anspruch 1 bis 3 mit einem Gehalt an freiem Quercetin (Aglykon) von höchstens 4 %, vorzugsweise höchstens 2 %.

5. Extrakt gemäß Anspruch 1 bis 4 mit einem Gehalt an Diphenylamin von < 0,1 %, vorzugsweise < 0,01 %.

6. Extrakt gemäß Anspruch 1 bis 5, wobei das organische Lösungsmittel zur Extraktion der Zwiebel n-Butanol ist.

7. Extrakt gemäß Anspruch 1 bis 6, umfassend die nachgeschalteten Schritte:
- Waschen der organischen Phase mit Wasser und
- Gewinnen der organischen Phase.

8. Extrakt gemäß Anspruch 1 bis 6, umfassend die nachgeschalteten Schritte:
- Waschen des Extraktes mit Wasser,
- Gewinnen und Verdampfen der organischen Phase,
- Extraktion des Rückstands mit einem unpolaren Lösungsmittel aus der Reihe der Alkane, Alkene, Ether, Ketone, Ester, insbesondere mit n-, iso-Hexan, n-, iso-Heptan, Methyl-tert.-butylether, Aceton und/oder überkritischem Kohlendioxid,
- Verwerfen der flüssigen organischen Phase bzw. des Extraktes, gewonnen mit überkritischem Kohlendioxid und
- Trocknen des Rückstands.

9. Extrakt gemäß Anspruch 1 bis 6, umfassend die nachgeschalteten Schritte:
- Waschen des Extraktes mit Wasser,
- Gewinnen, Einengen oder Verdampfen der organischen Phase,
- Umlösung des Rückstandes in Wasser,
- Extraktion des gelösten Rückstands mit einem organischen Lösungsmittel aus der Reihe der Alkane, Alkene, Ether, Ketone, Ester, insbesondere n-, iso-Hexan, n-, iso-Heptan, Methyl-tert.-butylether, Aceton und/oder überkritischem Kohlendioxid und
- Abtrennung und Verwerfen der organischen Phase.

10. Verfahren zur Herstellung eines Extraktes aus Zwiebeln gemäß Anspruch 1 bis 5, umfassend die Schritte:
- Extraktion frischer oder getrockneter Zwiebeln mit einem organischen Lösungsmittel aus der Reihe der Alkohole mit Ausnahme von Methanol und Ethanol, Ketone, Ester, Ether, insbesondere mit 1-, 2-Butanol, Aceton, Ethyl-, und/oder Methylacetat.
- Gewinnen der organischen Phase.

11. Verfahren gemäß Anspruch 10, wobei das organische Lösungsmittel zur Extraktion der Zwiebeln n-Butanol ist.

12. Verfahren gemäß Anspruch 10 oder 11, umfassend die nachgeschalteten Schritte:
- Waschen der organischen Phase mit Wasser und
- Gewinnen der organischen Phase.

13. Verfahren gemäß Anspruch 10 oder 11, umfassend die nachgeschalteten Schritte:
- Waschen des Extraktes mit Wasser,
- Gewinnen und Verdampfen der organischen Phase,
- Extraktion des Rückstands mit einem unpolaren Lösungsmittel aus der Reihe der Alkane, Alkene, Ether, Ketone, Ester, insbesondere mit n-, iso-Hexan, n-, iso-Heptan, Methyl-tert.-butylether, Aceton und/oder überkritischem Kohlendioxid,
- Verwerfen der flüssigen organischen Phase bzw. des Extraktes, gewonnen mit überkritischem Kohlendioxid und
- Trocknen des Rückstands.

14. Verfahren gemäß Anspruch 10 oder 11, umfassend die nachgeschalteten Schritte:
- Waschen des Extraktes mit Wasser,
- Gewinnen, Einengen oder Verdampfen der organischen Phase,
- Umlösung des Rückstandes in Wasser,
- Extraktion des gelösten Rückstands mit einem organischen Lösungsmittel aus der Reihe der Alkane, Alkene, Ether, Ketone, Ester, insbesondere n-, iso-Hexan, n-, iso-Heptan, Methyl-tert.-butylether, Aceton und/oder überkritischem Kohlendioxid und
- Abtrennung und Verwerfen der organischen Phase.

15. Verwendung der Extrakte gemäß der Ansprüche 1 bis 5 zur Herstellung von Arzneimitteln.

16. Verwendung der Extrakte gemäß der Ansprüche 1 bis 5 zur Herstellung von Nahrungsmitteln.

17. Verwendung der Extrakte gemäß der Ansprüche 1 bis 5 zur Herstellung von diätetischen Lebensmitteln und Nahrungsergänzungsmitteln.

18. Verwendung der Extrakte gemäß der Ansprüche 1 bis 5 zur Herstellung von Kosmetika.

19. Verwendung der Extrakte gemäß der Ansprüche 1 bis 5 zur Herstellung einer Zubereitung gemäß Ansprüchen 15 bis 18 mit antioxidativer, zell-, gefäß-, gewebs- und organprotektiver Wirkung.

20. Verwendung der Extrakte gemäß der Ansprüche 1 bis 5 zur Herstellung einer Zubereitung gemäß Ansprüchen 15 bis 18 zur Behandlung und Prophylaxe von Entzündungen.

21. Verwendung gemäß Anspruch 20, wobei die Entzündungen Nieren-, Blasen-, Harnwegs- und Prostataentzündungen im akuten und chronischen Stadium sind.

22. Verwendung gemäß Anspruch 20, wobei die Entzündungen Gelenkentzündungen und rheumatische Erkrankungen im akuten und chronischen Stadium sind.

23. Verwendung der Extrakte gemäß der Ansprüche 1 bis 5 zur Herstellung einer Zubereitung gemäß Ansprüchen 15 bis 18 zur Behandlung und Prophylaxe von Herz / Kreislauferkrankungen und Atherosklerose.

24. Verwendung der Extrakte gemäß der Ansprüche 1 bis 5 zur Herstellung einer Zubereitung gemäß Ansprüchen 15 bis 18 zur Behandlung und Prophylaxe von Diabetes mellitus begleitenden Erkrankungen, insbesondere Kataraktbildung, Neuropathien und Nierenschädigungen.

25. Verwendung der Extrakte gemäß der Ansprüche 1 bis 5 zur Herstellung einer Zubereitung gemäß Ansprüchen 15 bis 18 zur Behandlung und Prophylaxe von Demenzerkrankungen, Parkinson'scher Krankheit und Alzheimer'sche Krankheit.

## Claims

1. Extract from fresh or dried onions *(Allium cepa* L.), containing: at least 10 %, preferably 20 - 60 % of quercetin and/or quercetin derivatives, obtainable by a process comprising the steps:
- extraction of fresh or dried onions with an organic solvent from the alcohol series except for methanol and ethanol, ketones, esters, ethers, in particular with 1-butanol, 2-butanol, acetone, ethyl acetate and/or methyl acetate,
- isolation of the organic phase.

2. Extract according to Claim 1, containing: at least 10 %, preferably 20 - 60 % of quercetin glucosides.

3. Extract according to Claim 1 or 2 with a ratio of quercetin-3,4'-di-O-glucoside to quercetin-4'-O-glucoside of 0.5-2.

4. Extract according to Claims 1 to 3 with a content of free quercetin (aglycone) of at most 4 %, preferably at most 2%.

5. Extract according to Claims 1 to 4 with a content of diphenylamine of < 0.1 %, preferably < 0.01 %.

6. Extract according to Claims 1 to 5, wherein the organic solvent for the extraction of the onions is n-butanol.

7. Extract according to Claims 1 to 6, including the subsequent steps:
- washing of the organic phase with water and
- isolation of the organic phase.

8. Extract according to Claims 1 to 6, including the subsequent steps:
- washing of the extract with water,
- isolation and evaporation of the organic phase,
- extraction of the residue with a nonpolar solvent from the alkane series, alkenes, ethers, ketones and esters, in particular with n-hexane, isohexane, n-heptane, isoheptane, methyl tert.-butyl ether, acetone and/or supercritical carbon dioxide,
- rejection of the liquid organic phase or of the extract obtained with supercritical carbon dioxide and
- drying of the residue.

9. Extract according to Claims 1 to 6, including the subsequent steps:
- washing of the extract with water,
- isolation, concentration or evaporation of the organic phase,
- redissolution of the residue in water,
- extraction of the dissolved residue with an organic solvent from the alkane series, alkenes, ethers, ketones and esters, in particular n-hexane, isohexane, n-heptane, isoheptane, methyl tert.-butyl ether, acetone and/or supercritical carbon dioxide, and
- separation and rejection of the organic phase.

10. Process for the production of an extract from onions according to Claims 1 to 5, comprising the steps:
- extraction of fresh or dried onions with an organic solvent from the alcohol series except for methanol and ethanol, ketones, esters, ethers, in particular with 1-butanol, 2-butanol, acetone, ethyl acetate and/or methyl acetate
- isolation of the organic phase.

11. Process according to Claim 10, wherein the organic solvent for the extraction of the onions is n-butanol.

12. Process according to Claim 10 or 11, including the subsequent steps:
- washing of the organic phase with water and
- isolation of the organic phase.

13. Process according to Claim 10 or 11, including the subsequent steps:
- washing of the extract with water,
- isolation and evaporation of the organic phase,
- extraction of the residue with a nonpolar solvent from the alkane series, alkenes, ethers, ketones and esters, in particular with n-hexane, isohexane, n-heptane, isoheptane, methyl tert.-butyl ether, acetone and/or supercritical carbon dioxide,
- rejection of the liquid organic phase or of the extract obtained with supercritical carbon dioxide and
- drying of the residue.

14. Process according to Claim 10 or 11, including the subsequent steps:
- washing of the extract with water,
- isolation, concentration or evaporation of the organic phase,
- redissolution of the residue in water,
- extraction of the dissolved residue with an organic solvent from the alkane series, alkenes, ethers, ketones and esters, in particular n-hexane, isohexane, n-heptane, isoheptane, methyl tert.-butyl ether, acetone and/or supercritical carbon dioxide, and
- separation and rejection of the organic phase.

15. Use of the extracts according to Claims 1 to 5 for the production of medicaments.

16. Use of the extracts according to Claims 1 to 5 for the production of foodstuffs.

17. Use of the extracts according to Claims 1 to 5 for the production of dietetic foods and nutritional supplements.

18. Use of the extracts according to Claims 1 to 5 for the production of cosmetics.

19. Use of the extracts according to Claims 1 to 5 for the production of a preparation according to Claims 15 to 18 with antioxidant, cell, vascular, tissue and organ-protective action.

20. Use of the extracts according to Claims 1 to 5 for the production of a preparation according to Claims 15 to 18 for the treatment and prophylaxis of inflammation.

21. Use according to Claim 20, wherein the inflammation is kidney, bladder, urinary tract and prostate inflammation in the acute and chronic stage.

22. Use according to Claim 20, wherein the inflammation is joint inflammation and rheumatic diseases in the acute and chronic stage.

23. Use of the extracts according to Claims 1 to 5 for the production of a preparation according to Claims 15 to 18 for the treatment and prophylaxis of cardiovascular diseases and atherosclerosis.

24. Use of the extracts according to Claims 1 to 5 for the production of a preparation according to Claims 15 to 18 for the treatment and prophylaxis of diseases attendant on Diabetes mellitus, in particular cataract formation, neuropathies and kidney damage.

25. Use of the extracts according to Claims I to 5 for the production of a preparation according to Claims 15 to 18 for the treatment and prophylaxis of dementia diseases, Parkinson's disease and Alzheimer's disease.

## Revendications

1. Extrait d'oignons *(Allium cepa* L.) frais ou séchés, contenant au moins 10 %, de préférence 20-60 % de quercétine et/ou de dérivés de quercétine, pouvant être obtenu par un procédé comprenant les étapes suivantes :
- extraction d'oignons frais ou séchés par un solvant organique choisi dans la série des alcools à l'exception du méthanol et de l'éthanol, des cétones, des esters, des éthers, en particulier par le 1-butanol, le 2-butanol, l'acétone, l'acétate d'éthyle et/ou l'acétate de méthyle,
- collecte de la phase organique.

2. Extrait selon la revendication 1, contenant au moins 10 %, de préférence 20-60 % de quercétine-glucosides.

3. Extrait selon la revendication 1 ou 2, ayant un rapport du quercétine-3,4'-di-O-glucoside au quercétine-4'-O-glucoside de 0,5 - 2.

4. Extrait selon l'une quelconque des revendications 1 à 3, ayant une teneur en quercétine libre (aglycone) d'au maximum 4 %, de préférence d'au maximum 2 %.

5. Extrait selon l'une quelconque des revendications 1 à 4, ayant une teneur en diphénylamine de < 0,1 %, de préférence de < 0,01 %.

6. Extrait selon l'une quelconque des revendications 1 à 5, dans lequel le solvant organique pour l'extraction des oignons est le n-butanol.

7. Extrait selon l'une quelconque des revendications 1 à 6, comprenant les étapes subséquentes :
- lavage de la phase organique avec de l'eau et
- collecte de la phase organique.

8. Extrait selon l'une quelconque des revendications 1 à 6, comprenant les étapes subséquentes suivantes :
- lavage de l'extrait avec de l'eau,
- collecte et évaporation de la phase organique,
- extraction du résidu par un solvant non polaire choisi dans la série des alcanes, alcènes, éthers, cétones, esters, en particulier par le n-hexane, l'isohexane, le n-heptane, l'isoheptane, l'oxyde de méthyle et de tert-butyle, l'acétone et/ou le dioxyde de carbone supercritique,
- élimination de la phase organique liquide ou de l'extrait obtenu avec du dioxyde de carbone supercritique et
- séchage du résidu.

9. Extrait selon l'une quelconque des revendications 1 à 6, comprenant les étapes subséquentes :
- lavage de l'extrait avec de l'eau,
- collecte, concentration ou évaporation de la phase organique,
- dissolution du résidu dans de l'eau,
- extraction du résidu dissous par un solvant organique choisi dans la série des alcanes, alcènes, éthers, cétones, esters, en particulier le n-hexane, l'isohexane, le n-heptane, l'isoheptane, l'oxyde de méthyle et de tert-butyle, l'acétone et/ou le dioxyde de carbone supercritique et
- séparation et élimination de la phase organique.

10. Procédé pour la préparation d'un extrait d'oignons selon l'une quelconque des revendications 1 à 5, comprenant les étapes suivantes :
- extraction d'oignons frais ou séchés par un solvant organique choisi dans la série des alcools à l'exception du méthanol et de l'éthanol, des cétones, des esters, des éthers, en particulier par le 1-butanol, le 2-butanol, l'acétone, l'acétate d'éthyle et/ou l'acétate de méthyle,
- collecte de la phase organique.

11. Procédé selon la revendication 10, dans lequel le solvant organique pour l'extraction des oignons est le n-butanol.

12. Procédé selon la revendication 10 ou 11, comprenant les étapes subséquentes :
- lavage de la phase organique avec de l'eau et
- collecte de la phase organique.

13. Extrait selon la revendication 10 ou 11, comprenant les étapes subséquentes :
- lavage de l'extrait avec de l'eau,
- collecte et évaporation de la phase organique,
- extraction des résidus par un solvant organique choisi dans la série des alcanes, alcènes, éthers, cétones, esters, en particulier par le n-hexane, l'isohexane, le n-heptane, l'isoheptane, l'oxyde de méthyle et de tert-butyle, l'acétone et/ou le dioxyde de carbone supercritique,
- élimination de la phase organique liquide ou de l'extrait obtenu avec du dioxyde de carbone supercritique et
- séchage du résidu.

14. Procédé selon la revendication 10 ou 11, comprenant les étapes subséquentes suivantes :
- lavage de l'extrait avec de l'eau et
- collecte, concentration ou évaporation de la phase organique,
- dissolution du résidu dans de l'eau,
- extraction du résidu dissous par un solvant organique choisi dans la série des alcanes, alcènes, éthers, cétones, esters, en particulier le n-hexane, l'isohexane, le n-heptane, l'isoheptane, l'oxyde de méthyle et de tert-butyle, l'acétone et/ou le dioxyde de carbone supercritique et
- séparation et élimination de la phase organique.

15. Utilisation des extraits selon les revendications 1 à 5, pour la fabrication de médicaments.

16. Utilisation des extraits selon les revendications 1 à 5, pour la fabrication de produits alimentaires.

17. Utilisation des extraits selon les revendications 1 à 5, pour la fabrication d'aliments diététiques et de compléments alimentaires.

18. Utilisation des extraits selon les revendications 1 à 5, pour la fabrication de cosmétiques.

19. Utilisation des extraits selon les revendications 1 à 5, pour la fabrication d'une préparation selon les revendications 15 à 18, à effet antioxydant, cytoprotecteur, vasoprotecteur et protecteur des tissus et des organes.

20. Utilisation des extraits selon les revendications 1 à 5, pour la fabrication d'une préparation selon les revendications 15 à 18, destinée au traitement et à la prophylaxie d'inflammations.

21. Utilisation selon la revendication 20, les inflammations étant des inflammations des reins, de la vessie, des voies urinaires et de la prostate, au stade aigu et au stade chronique.

22. Utilisation selon la revendication 20, les inflammations étant des inflammations des articulations et des inflammations rhumatismales, au stade aigu et au stade chronique

23. Utilisation des extraits selon les revendications 1 à 5, pour la fabrication d'une préparation selon les revendications 15 à 18, destinée au traitement et à la prophylaxie de maladies cardiovasculaires et de l'athérosclérose.

24. Utilisation des extraits selon les revendications 1 à 5, pour la fabrication d'une préparation selon les revendications 15 à 18, destinée au traitement et à la prophylaxie de maladies associées au diabète sucré, en particulier la cataracte, les neuropathies et les lésions rénales.

25. Utilisation des extraits selon les revendications 1 à 5, pour la fabrication d'une préparation selon les revendications 15 à 18, destinée au traitement et à la prophylaxie de démences, de la maladie de Parkinson et de la maladie d'Alzheimer.
